**Europäisches Patentamt**

**European Patent Office**

**Office européen des brevets**

(19)

(11) Veröffentlichungsnummer: **0 150 197**
**B1**

(12)

# EUROPÄISCHE PATENTSCHRIFT

(45) Veröffentlichungstag der Patentschrift:
**13.05.87**

(51) Int. Cl.⁴: **C 12 M 1/12**

(21) Anmeldenummer: **84902701.6**

(22) Anmeldetag: **28.06.84**

(86) Internationale Anmeldenummer:
**PCT/EP 84/00197**

(87) Internationale Veröffentlichungsnummer:
**WO 85/00183 (17.01.85 Gazette 85/2)**

(54) **VORRICHTUNG ZUR KONTINUIERLICHEN FERMENTATION MIT GLEICHZEITIGER FRAKTIONIERTER STOFFWECHSELPRODUKTABTRENNUNG.**

(30) Priorität: **28.06.83 DE 3323205**

(43) Veröffentlichungstag der Anmeldung:
**07.08.85 Patentblatt 85/32**

(45) Bekanntmachung des Hinweises auf die Patenterteilung:
**13.05.87 Patentblatt 87/20**

(84) Benannte Vertragsstaaten:
**AT BE CH DE FR GB LI LU NL SE**

(56) Entgegenhaltungen:
**CH - A - 4 259**
**FR - A - 934 939**
**FR - A - 2 430 451**
**GB - A - 1 113 630**
**US - A - 3 472 765**

(73) Patentinhaber: **Carl Schleicher & Schüll GmbH & Co. KG, Grimsehlstrasse 23, D-3352 Einbeck (DE)**

(72) Erfinder: **KOHLHEB, Robert, Andershäuser Strasse 15, D-3352 Einbeck (DE)**

(74) Vertreter: **Jaeger, Klaus, Dipl.-Chem. Dr. et al, JAEGER & PARTNER Patentanwälte Pippinplatz 4a, D-8035 Gauting (DE)**

ACTORUM AG

## Beschreibung

Die Erfindung betrifft eine Vorrichtung zur kontinuierlichen Fermentation eines inokulierten flüssigen Substrats mit gleichzeitiger kontinuierlicher fraktionierter Abtrennung der im Fermentationsgemisch mikrobiologisch gebildeten Stoffwechselprodukte.

In der amerikanischen Patentschrift Nr. 3 472 765 wird ein Verfahren zur biologischen Abwasserreinigung beschrieben, das sinngemäss auch auf Fermentationsprozesse übertragen werden kann. Bei diesem Verfahren wird das feste Partikel enthaltende Abwasser in einen den geeigneten Mikroorganismus enthaltenden Reaktor eingeleitet und in einem ersten Kreislauf umgewälzt. In den Reaktor kann Sauerstoff eingeleitet werden und gasförmige Nebenprodukte, z.B. $CO_2$, abgeleitet werden. Dem Reaktor wird kontinuierlich gerade so viel Reaktionssuspension entnommen, dass das Reaktionsvolumen im Reaktor konstant bleibt. Das entnommene Reaktionsgemisch bestehend aus Mikroorganismus, den Stoffwechselprodukten des Mikroorganismus, festen, nicht umgewandelten Partikeln und Wasser wird in einem zweiten Kreislauf, in den ein Membranfilter integriert ist, mittels einer Pumpe umgewälzt.

Die Strömungsgeschwindigkeit in dem zweiten Kreislauf ist erheblich grösser als die Strömungsgeschwindigkeit im ersten Kreislauf, um Turbulenzen auf der Membran des Membranfilters zum Zwecke einer effizienten und unproblematischen Stofftrennung zu erzeugen. Beide Kreisläufe sind in der Art miteinander gekoppelt, dass die die Membran verlassende konzentrierte Suspension partiell in den Reaktor rückgeführt werden kann. Dem Membranfilter kann kontinuierlich das aufgearbeitete Abwasser entnommen werden. Durch Hintereinanderschalten zweier Membranen kann eine Fraktionierung des Abwassers erreicht werden.

In der französischen Druckschrift FR-A-2 430 451 ist ein ähnliches Verfahren zur Abwasseraufbereitung beschrieben. Es unterscheidet sich von dem zuvor beschriebenen Verfahren primär dadurch, dass lediglich ein Kreislauf zwischen dem Reaktor und dem Membranfilter zur Ultrafiltration vorgesehen ist. Es können grundsätzlich beliebige Filtrationsgeräte, die zur Ultrafiltration verwendbar sind, benutzt werden, einschliesslich Filtrationsgeräten mit spiralförmig aufgerollter Membran.

Angesichts dieses Standes der Technik liegt der Erfindung die Aufgabe zugrunde, eine Vorrichtung zur kontinuierlichen Fermentation mit gleichzeitiger fraktionierter Stoffwechselproduktabtrennung zu schaffen, die weniger Raum beansprucht und doch den für die grosstechnische Produktion erforderlichen Durchsatz ermöglicht.

Zur Lösung dieser Aufgabe wird eine Vorrichtung mit den im Anspruch 1 genannten Merkmalen geschaffen.

Die Vorrichtung ist durch die Integration der Filtrationsmembran in den Bioreaktor gekennzeichnet, wobei die Membran auf einen die Zu- und Ablaufrohre enthaltenden Wickelkern aufgerollt ist. Das aus inokuliertem Substrat, bereits fermentiertem Substrat und den gebildeten Stoffwechselprodukten neben Biomasse und gegebenenfalls anderen Komponenten bestehende Fermentationsgemisch wird im Kreislauf durch den Bioreaktor geführt und innerhalb dieses Kreislaufs einer kontinuierlichen Dünnschichtfermentation ausgesetzt, wobei das Fermentationsgemisch zu diesem Zweck in dünner Schicht, vorzugsweise als dünner Flüssigkeitsfilm unter gleichzeitiger Druckbeaufschlagung und, bei aeroben Prozessen, unter gleichzeitiger Belüftung, über die Oberfläche einer Filtrationsmembran geführt wird, deren Porengrösse so gewählt ist, dass unter den eingestellten Verfahrensbedingungen die mikrobiologisch gebildeten Stoffwechselprodukte durch die Membran hindurchtreten und filtratseitig gesammelt und abgezogen werden, während die Mikroorganismen im Fermentationsgemisch-Kreislauf verbleiben. Dabei kann insbesondere bei der vorzugsweise erfolgenden Verwendung von Membranfilter-Spiralwickelelementen, die vom Fermentationsgemisch tangential, radial von innen nach aussen oder von aussen nach innen fortschreitend durchströmt werden, bei kleinem Bauvolumen der Reaktorgefässe eine so grosse Membranoberfläche zur Verfügung gestellt werden, dass das auf diese Weise kontinuierlich geführte biotechnische Fermentationsverfahren eine Produktivität aufweist, die den kommerziellen Anforderungen technischer Produktionsweisen genügt.

Die Membran, die die dünne strömende Schicht des Fermentationsgemisches trägt und durch die hindurch die Stoffwechselprodukte nach Molekulargewichten fraktioniert selektiv abgetrennt werden, ist vorzugsweise hydrophob. Durch die Hydrophobie der Membran werden ein Festsetzen von Mikroorganismen auf der Membran, ein übermässiges Belegen der überströmten Membranoberfläche mit anderen Bestandteilen des Fermentationsgemisches verhindert und wird die Abtrennung der Stoffwechselprodukte signifikant verbessert.

Als Werkstoff für die aus den vorstehend genannten Gründen vorzugsweise hydrophobe Membran werden vorzugsweise fluorierte Kohlenwasserstoffpolymere eingesetzt, vor allem Polytetrafluorethylen.

Entsprechend einer bevorzugten Ausgestaltung der Erfindung ist dem Membranreaktor ein mit einer Heizung und einem Filter ausgestatteter Sterilisierungsmodul vorgeschaltet. Hier kann das Substrat auf Temperaturen von beispielsweise 130°C aufgeheizt und von Verunreinigungen steril abfiltriert werden. Das heisse Filtrat wird vorzugsweise durch ein Kühlaggregat auf die Temperatur des Fermentationsgemisches abgekühlt und mittels geeigneter Zuleitung in den Membranreaktor eingeleitet.

Die nach der Sterilisation dem erhitzten sterilisierten Substrat entzogene Wärmemenge kann zum Vorwärmen des Substrats verwendet werden, wenn es als Kühlflüssigkeit für das erwähnte Kühlaggregat verwendet wird. In diesem Fall empfiehlt es sich, einen vorgeschalteten Vorratsspeicher vorzusehen, der als Ringleitung mit integriertem Vorratsbehälter ausgebildet ist und in dem das Substrat unter Passieren des Kühlaggregats bzw. Wärmeaustauschers im Kreislauf geführt wird.

Vorzugsweise entspricht das Volumen des Vorratsbehälters etwa dem zweifachen oder dreifachen Tagesbedarf des kontinuierlichen Fermentationsprozesses, so dass die kontinuierlich arbeitende Anlage nur alle zwei oder drei Tage mit einer entsprechenden Zwei- bis Dreitagesmenge frischem Rohsubstrat beschickt zu werden braucht.

Nach einer weiteren Ausgestaltung der Erfindung wird die Menge bzw. wird der Volumenstrom des aus dem Speicherkreislauf über das Sterilisationsmodul und ein Inokulationsmodul in den Fermentationsgemischkreislauf überführten Substrats unter Verwendung des im Fermentationsgemischkreislauf umlaufenden Flüssigkeitsvolumens als Führungsgrösse geregelt. Nach einer bevorzugten Ausgestaltung der Erfindung erfolgt die Abtastung der Führungsgrösse vorzugsweise durch einen Füllstandsmesser, der den Flüssigkeitsspiegel in einem unteren Bodenbereich des zur Durchführung der Fermentation und Stoffabtrennung benutzten Membranreaktors abtastet. Diese, beispielsweise durch einen Schwimmer abgetasteten Niveauhöhen können dann unmittelbar oder unter Zwischenschaltung einer Signalstrecke zum Stellen eines in die Substratzulaufleitung eingeschalteten Ventils dienen. Die Substratversorgung und die kontinuierliche Fermentation mit der kontinuierlich fraktionierten selektiven Produktabtrennung sind also auf diese Weise zu einem integrierten und selbstregulierenden Prozess zusammengefasst.

Nach einer noch weiteren Ausgestaltung der Erfindung ist schliesslich vorgesehen, dass die Membran im Fermentationsreaktor oder Bioreaktor auf der Filtratseite der Membran mit steriler Druckluft beaufschlagt werden kann. Die Sterilisation der hierzu verwendeten Druckluft erfolgt dabei vorzugsweise durch eine Membranfiltration komprimierter Umgebungsluft, die gegebenenfalls mit Sauerstoff angereichert sein kann. Die hierzu verwendeten Membranfilter sind vorzugsweise ebenfalls hydrophob, bestehen vorzugsweise ebenfalls aus fluorierten Kohlenwasserstoffpolymeren, insbesondere Polytetrafluorethylen, und haben eine Porengrösse, die zumindest Bakterien, vorzugsweise auch Viren und Pyrogene, zurückhält.

Diese sterile Druckluft kann einerseits von der Filtratseite her entweder durch die zur Produktfiltration benutzte Membran hindurch oder durch andere, nur diesem Zweck dienende parallele Membranen hindurch in den oberen Teil des Membranreaktors und damit in den über dem Flüssigkeitsspiegel des Fermentationsgemisches im Membranreaktor gebildeten Druckgasraum eingedrückt werden, und kann andererseits, bei geringerer Druckbeaufschlagung, dem ständigen Spülen und Belüften der filtratseitigen Membranoberfläche dienen.

Die Erfindung ist im folgenden anhand eines Ausführungsbeispiels in Verbindung mit den Zeichnungen näher erläutert.

Es zeigen:

Fig. 1 in schematischer Darstellung das Blockschaltbild einer Speicher- und Sterilisationsanlage für das flüssige Substrat;

Fig. 2 das Blockschaltbild einer Vorrichtung zur Durchführung der kontinuierlichen Fermentation mit kontinuierlichem Zulauf des sterilen Substrats und kontinuierlichem Produktablauf;

Fig. 3 im Axialschnitt einen Membranreaktor zur Durchführung der kontinuierlichen Fermentation mit kontinuierlicher Produktabtrennung;

Fig. 4 einen Schnitt nach IV-IV in Fig. 3;

Fig. 5 einen Schnitt nach V-V in Fig. 3;

Fig. 6 im Axialschnitt einen beheizbaren Filter zum Sterilfiltrieren des Substrats.

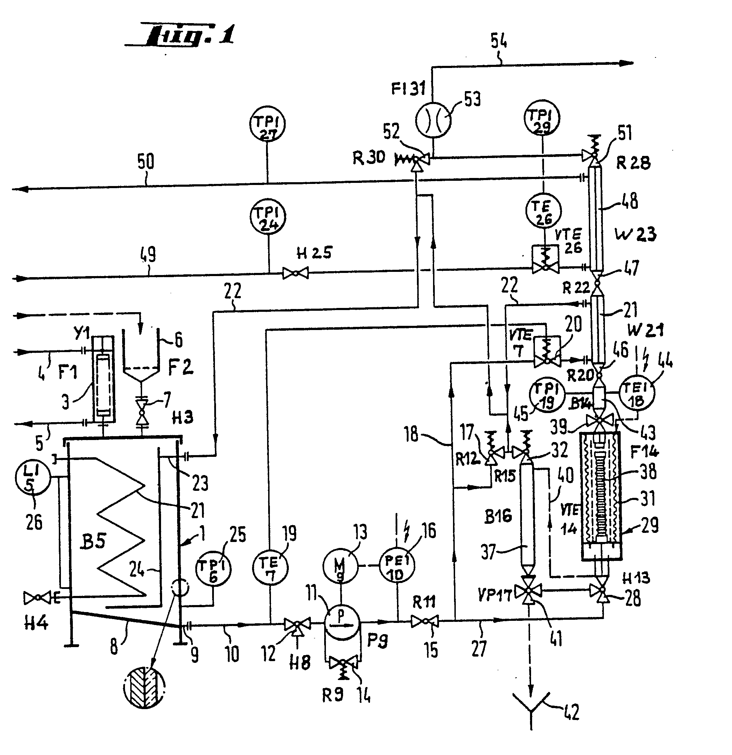

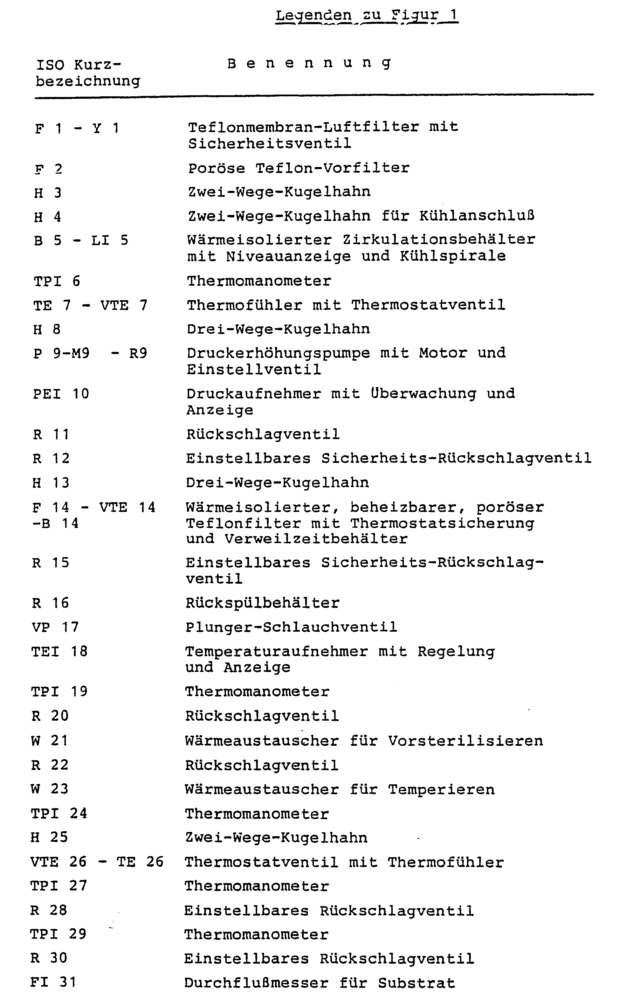

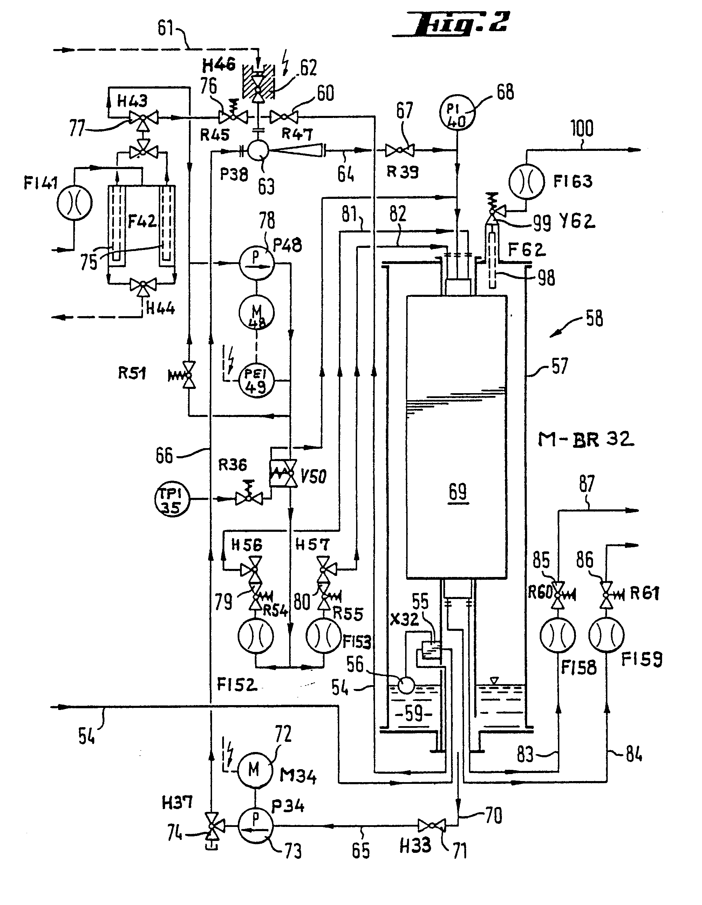

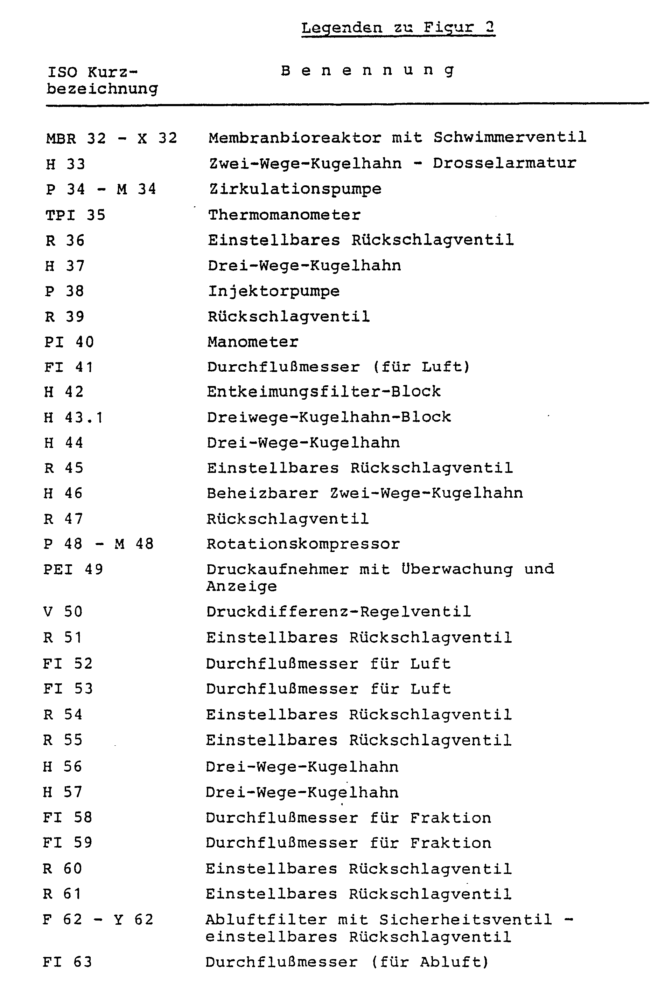

In den Fig. 1 und 2, denen jeweils eine Legende nach ISO beigefügt ist, sind der Substratvorbereitungsteil und der Fermentationskreislauf einer integrierten Anlage zur kontinuierlichen Herstellung eines erwärmten sterilen Substrats und zur kontinuierlichen Fermentation dargestellt.

Das Hauptvolumen der in der Fig. 1 schematisch dargestellten Anlage zum Speichern und Sterilisieren des Substrats ist in einem grossen Edelstahlbehälter 1 enthalten, der zur Temperaturkontrolle mit einer Kühlvorrichtung 2 ausgerüstet ist. Die Belüftung des Vorratsbehälters 1 erfolgt über einen PTFE-Membranfilter 3, der über eine Zuleitung 4 mit Umgebungsdruckluft beaufschlagt wird. Die Abluft verlässt den Filter über die Abluftleitung 5. Die Porengrösse der Luftfiltermembran liegt im Bereich von 0,01 bis 0,5 μm, vorzugsweise bei 0,45 μm, so dass also in der zugeführten Druckluft enthaltene Bakterien zurückgehalten werden. Das verwendete Luftfiltermaterial ist hydrophob, so dass sich weder innen noch aussen auf der Filtermembran ein zusammenhängender Flüssigkeitsfilm ausbilden kann. Das Kondenswasser, das sich auf dem Filter bildet, wird aussen durch die Abluftleitung 5 abgeführt und kann innen in den Behälter 1 zurücklaufen. Der Filter 3 ist aus Sicherheitsgründen mit einem Druckausgleichsventil versehen. Ein solches Ventil ist insbesondere für eine erforderlichenfalls rasche Entleerung des Speicherbehälters 1 erforderlich.

Am Kopf des Speicherbehälters 1 ist weiterhin ein Vorfilter 6 vorgesehen, durch den hindurch über einen Kugelhahn 7 frisch bereitetes Substrat in den Speicherbehälter 1 nachgefüllt werden kann. Der Vorfilter 6 ist ein poröser PTFE-Filter mit einer Porengrösse von 10 μm.

Über den Vorfilter 6 wird der Vorratsbehälter 1 diskontinuierlich nachgefüllt, vorzugsweise in einer Nachfüllmenge, die einem Zweitagesbedarf der nachgeschalteten Fermentationsanlage entspricht.

Zur Vermeidung von Schlammablagerungen ist der Vorratsbehälter 1 mit einem schrägen Boden 8 versehen. An der tiefsten Stelle des Behälters 1 ist ein Ablaufstutzen 9 vorgesehen, an dem eine Zweigleitung 10 zu einer Pumpe 11 angeschlossen ist. Vor der Pumpe 11 ist in die Leitung 10 ein Dreiwegehahn 12 eingeschaltet, der für die Wartung und Reinigung der Anlage erforderlich ist. Die Druckpumpe 11 wird von einem Elektromotor 13 beaufschlagt und ist in dem hier beschriebenen Ausführungsbeispiel als Edelstahl-Trennschieberpumpe mit eingebautem federbelastetem Einstellventil 14 ausgelegt. Die über ein Rückschlagventil 15 abgesicherte Druckleitung wird durch einen Druckwächter 16 überwacht. Die eigentliche Druckeinstellung im Druckteil der Anlage erfolgt dabei über ein einstellbares Sicherheits-Rückschlagventil 17, das gleichzeitig das Hauptsicherheitsventil der gesamten Anlage ist. Der durch die Pumpe 11 im Kreislauf geführte umgewälzte Teil des Substrats gelangt über eine Druckleitung 18 und ein durch den Thermofühler 19 gesteuertes Stellventil 20 auf einen Wärmetauscher 21, in dem es erwärmt und von dem es anschliessend über eine Rücklaufleitung 22 zu einem Rücklauf-Anschlussstutzen 23 des Behälters 1 zurückgeführt wird. Der Rücklauf-Anschlussstutzen 23 ist innen im Vorratsbehälter 1 mit einem Tauchrohr 24 verbunden, durch den das im Kreislauf rückgeführte erwärmte Substrat am Boden des Speicherbehälters 1 in das Vorratsvolumen des Substrats eingeleitet wird. Dabei wird die Temperatur im Behälter durch ein Thermometer 25 und der Flüssigkeitsstand im Behälter 1 durch einen Niveauwächter 26 überwacht.

Das auf diesem Wege unter Beaufschlagung durch die Pumpe 11 im Speicherkreislauf 1, 10, 18, 21, 22 gespeicherte und umgewälzte Vorratssubstrat wird durch Erwärmung im Wärmeaustauscher 21, Kühlung durch den Kühler 2 und primäre Steuerung durch das Stellventil 20 auf eine Temperatur von 80 °C eingeregelt.

Von der druckseitigen Kreislaufleitung 18 zweigt eine Verbindungsleitung 27 ab, über die das vorerwärmte bakterienfreie Substrat über einen Dreiwegehahn 28 auf ein heizbares Sterilisationsfiltermodul 29 überführt wird.

Ein Ausführungsbeispiel für ein solches Sterilisationsfiltermodul 29 ist im Axialschnitt in der Fig. 6 dargestellt. Das eigentliche Filter ist eine poröse PTFE-Filterkerze 30, die aussen zylindrisch mit einer elektrischen Widerstandsheizung 31 umgeben ist.

Bei Inbetriebnahme der Anlage wird das in Fig. 1 gezeigte einstellbare Rückschlagventil 32 geöffnet. Dies ermöglicht ein rasches Aufheizen des im Vorratsbehälter 1 gespeicherten Substrats auf die Solltemperatur von 80 °C. Wenn diese Temperatur erreicht ist, wird das Ventil 32 geschlossen, bzw. damit auf seine Sicherheitsfunktion umgeschaltet. Über den Dreiwegehahn 28 gelangt dann das auf 80 °C vorgewärmte Substrat über den Einlass 33 (Fig. 6) in den Druckbehälter 34 des Sterilisationsfiltermoduls 29. Dort wird das Substrat durch die Heizung 31 auf eine Temperatur von bis zu 130 °C erhitzt. Das erhitzte Substrat tritt dann durch die PTFE-Filterkerze, die eine Porengrösse von 1 µm aufweist, in den Filtratraum 35 des Moduls 29 über und verlässt diesen durch den Ablaufstutzen 36.

Zur Reinigung der Filterkerze ist im Filtratraum 35 ein mit einem komprimierbaren Gas gefüllter Faltenbalg 38 angeordnet, der unter normalen Betriebsbedingungen durch den im Filtratraum herrschenden Flüssigkeitsdruck komprimiert ist. Zur Rückspülung wird der Dreiwegehahn 28 kurzfristig von der Betriebsstellung in eine Rückspülstellung umgestellt. Dabei läuft das unter Druck stehende und auslassseitig durch ein Sicherheitsventil 39 abgesicherte Filtrat unter Druckentlastung über eine Rückspülleitung 40 in einen Rückspülbehälter 37. Dabei wird das Ausdrücken des Rückspülsubstrats aus dem Filtratraum durch den sich unter dem Druckabfall expandierenden Faltenbalg 38 unterstützt.

Aus dem Rückspülbehälter 37 gelangt das Rückspülsubstrat über ein sich periodisch öffnendes Ablaufventil 41 in den Abwasserkanal 42. Dabei wird das als Membrankolbenventil ausgebildete Ablaufventil 41 durch das von der Pumpe 11 mit Druck beaufschlagte Substrat gestellt.

Beim erneuten Umstellen des Dreiwegehahns 28 aus der Rückspülstellung in die Betriebsstellung wird der Druck im Filtratraum wieder aufgebaut, so dass der Faltenbalg 38 erneut zusammengedrückt wird.

Wenn das Filtrat die für die Sterilisation vorbestimmte Temperatur erreicht hat, öffnet das Thermostatsicherheitsventil 39, so dass das auf die Solltemperatur erhitzte, filtrierte und sterilisierte Substrat aus dem Filtratraum austreten und in einen Verweilzeitbehälter 43 überführt werden kann. In diesem Behälter verweilt das erhitzte Substrat so lange, bis alle gegebenenfalls noch im Substrat verbliebenen unerwünschten Mikroorganismen abgetötet sind, das Substrat also vollständig sterilisiert ist. Dabei wird die Einhaltung der erforderlichen Sterilisationstemperatur im Verweilzeitbehälter 43 durch einen Temperaturregler 44 geregelt und durch ein Thermometer 45 überwacht. Dabei steuert der Regler 44 die Temperatur der Heizung 31 im Sterilisationsfiltermodul 29.

Über ein Rückschlagventil 46 gelangt das sterilisierte erhitzte Substrat aus dem Verweilzeitbehälter 43 in den Wärmeaustauscher 21, in dem es durch das umgewälzte vorgewärmte Substrat auf ungefähr 85 °C abgekühlt wird.

Aus dem Wärmeaustauscher 21 gelangt das auf ca. 85 °C abgekühlte Substrat über ein weiteres Rückschlagventil 47 auf einen zweiten Wärmeaustauscher 48, und wird mittels Kühlwasser, das über Kühlwasserleitungen 49, 50 geführt wird, genau auf die Temperatur eingestellt, auf die das Fermentationsgemisch im nachgeschalteten Fermentationskreislauf (vgl. unten) eingeregelt ist. Dabei wird die Kühlwassertemperatur in üblicher Weise durch Temperaturwächter und Thermostaten auf die erforderliche Temperatur eingestellt. Über ein Sicherheitsventil 51, ein einstellbares Sicherheits-Rückschlagventil 52 und einen Durchflusswächter 53 gelangt das vollkommen sterilisierte und druckbeaufschlagte Substrat dann in die Zulaufleitung 54 zur Kreislaufleitung im Fermentationsprozess.

In die Verbindungsleitung 54 ist ein Stellventil 55 eingeschaltet, das von einem Schwimmer 56 gestellt wird, der auf der Oberfläche eines im Bodenbereich des Reaktorgehäuses 57 des Membranreaktors 58 als Puffer- und Steuerreservoir eingerichtet ist.

Auf diese Weise gelangt das sterile Substrat unter Führung durch den Spiegel des am Boden des Reaktorgefässes 57 angesammelten Fermentationsgemisches 59 über ein Rückschlagventil 60, erforderlichenfalls unter Zusatz von frischem Inoculum, das über eine Zulaufleitung 61 und ein beheiztes Ventil 62 zugeführt wird, in eine Injektorpumpe 63, die in der Fermentationsgemischkreisleitung 64 zur Druckbeaufschlagung des im Kreislauf geführten Fermentationsgemisches eingeschaltet ist. Unter dem Fermentationgemisch wird dabei das gesamte Gemisch verstanden, das in dem Druckast 64, dem entspannten Abschnitt 65 und dem vorgespannten Abschnitt 66 der Kreislaufleitung umläuft und aus mit Inoculum versetztem frischen Substrat, fermentierten Substrat, Stoffwechselprodukten, Biomasse und gegebenenfalls weiteren Zusatzstoffen zusammengesetzt ist.

Je nach Art des Fermentationsprozesses und des Membranreaktors wird das Fermentationsgemisch durch die Injektorpumpe auf einen Druck von bis zu 100 bar Überdruck, in der Regel vorzugsweise auf einen Druck im Bereich von 1 bis 5 bar Überdruck gebracht und mit dieser Druckbeaufschlagung über ein Rückschlagventil 67 auf den Membranreaktor 58 gegeben. Dabei wird der in der Druckleitung 64 herrschende Druck durch ein Manometer 68 überwacht. Nach Überströmen der Membranoberfläche, wobei die Stoffwechselprodukte abgetrennt und der Fermentationsprozess fortgeführt werden, läuft das Fermentationsgemisch vom Membranfilterelement 69 in Reaktorgehäuse 57 in das Bodenreservoir des Reaktorgehäuses ab. Von dort gelangt es über die Ablaufleitung 70 und ein Drosselventil 71 in den entspannten Ast 65 der Kreislaufleitung für das Fermentationsgemisch. Über eine von einem Elektromotor 72 beaufschlagte Umwälzpumpe 73 und einen zu Wartungs- und Reinigungszwecken erforderlichen Dreiwegehahn 74 gelangt das zirkulierende Fermentationsgemisch über den Druckast 66 der Umwälzleitung wieder zurück zur Injektorpumpe 63. Die im Flüssigkeitsreaktor 58 für die Fermentationsprozesse benötigte sterile Druckluft wird über PTFE-Filter 75 mit hydrophoben Membranen und einer Porengrösse, die Bakterien zurückhält, und über einen Verteilerhahn 77 entweder über ein einstellbares Rückschlagventil 76 direkt in das Fermentationsgemisch eingedrückt oder über eine Luftpumpe 78 und einstellbare Rückschlagventile 79, 80 mit nachgeschalteten Druckluftleitungen 81, 82 in den Reaktor 58 gegeben.

Die filtratseitig aus dem Reaktor abgezogenen Stoffwechselproduktfraktionen gelangen über die Ablaufleitungen 83, 84 und die einstellbaren Rückschlagventile 85, 86 an die Entnahmeanschlüsse 87 bzw. 88.

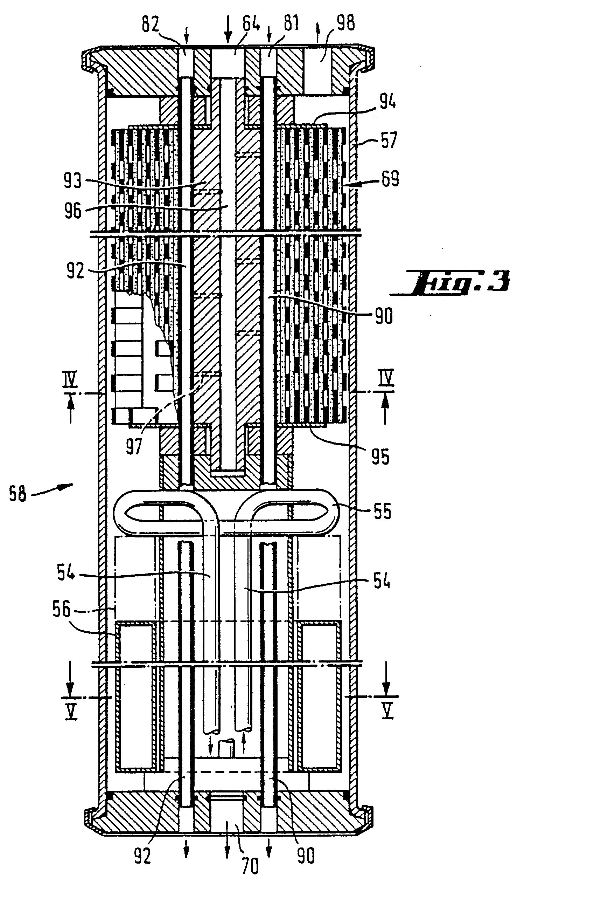

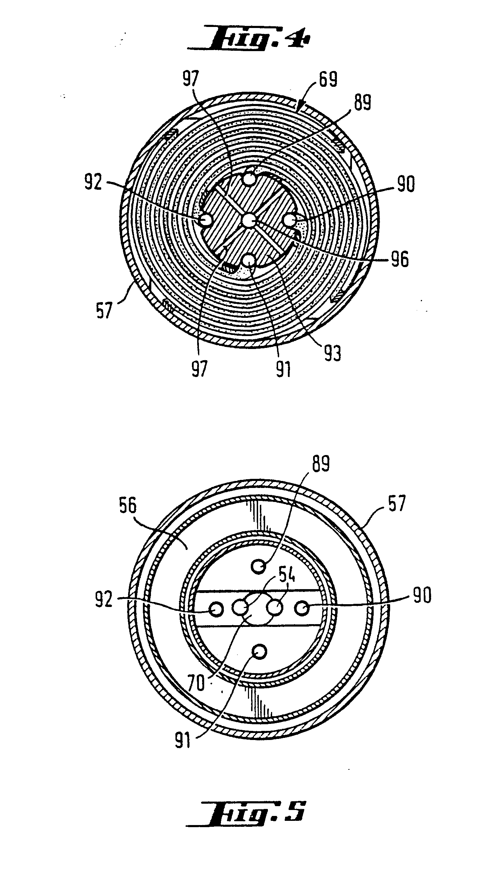

Ein Ausführungsbeispiel für einen solchen Membranreaktor 58 ist in den Fig. 3 bis 5 gezeigt.

Die von dem druckbeaufschlagten Fermentationsgemisch überströmte Membran ist als Spiralwickelelement 69 ausgebildet. Das Wickelelement 69 besteht aus mehreren, in dem hier gezeigten Ausführungsbeispiel 4 Membrantaschen aus hydrophoben PTFE-Membranen, die zu Ablaufröhren 89, 90, 91 bzw. 92 geöffnet sind, die axial verlaufend in den Aussenmantel des Wickelkerns 93 des Spiralwickelelements eingearbeitet sind.

Die Membranen bestehen in dem hier beschriebenen Ausführungsbeispiel aus PTFE. Gleicherweise können jedoch auch andere Membranwerkstoffe verwendet werden, insbesondere Membranen, und zwar vorzugsweise hydrophobe Membranen, aus Celluloseacetat, aus Polyamid sowie aus Polysulfonen. Je nach dem beabsichtigten Anwendungsgebiet und nach der abzutrennenden Stoffwechselproduktfraktion können die Membranen Porengrössen vom Mikrofiltrationsbereich bis zum Hyperfiltrationsbereich haben, also Porengrössen im Bereich von 0,1 bis 0,001 µm. Dabei brauchen auch nicht alle Membrantaschen aus Membranen mit gleichen Porengrössen bestehen, sondern können die Membrantaschen auch unterschiedliche Porengrössen aufweisen, so dass unterschiedliche Fraktionen der Stoffwechselprodukte separat voneinander abgetrennt werden können, da sich jede einzelne Membrantasche in ein separates Ableitungsröhrchen 89 bis 92 öffnet und die Produktfraktionen aus diesen Ablaufröhrchen selektiv entnommen werden können. In dem in Fig. 3 gezeigten Ausführungsbeispiel sind die Ableitungsröhrchen aus dem stirnseitigen Boden des Membranreaktors herausgeführt.

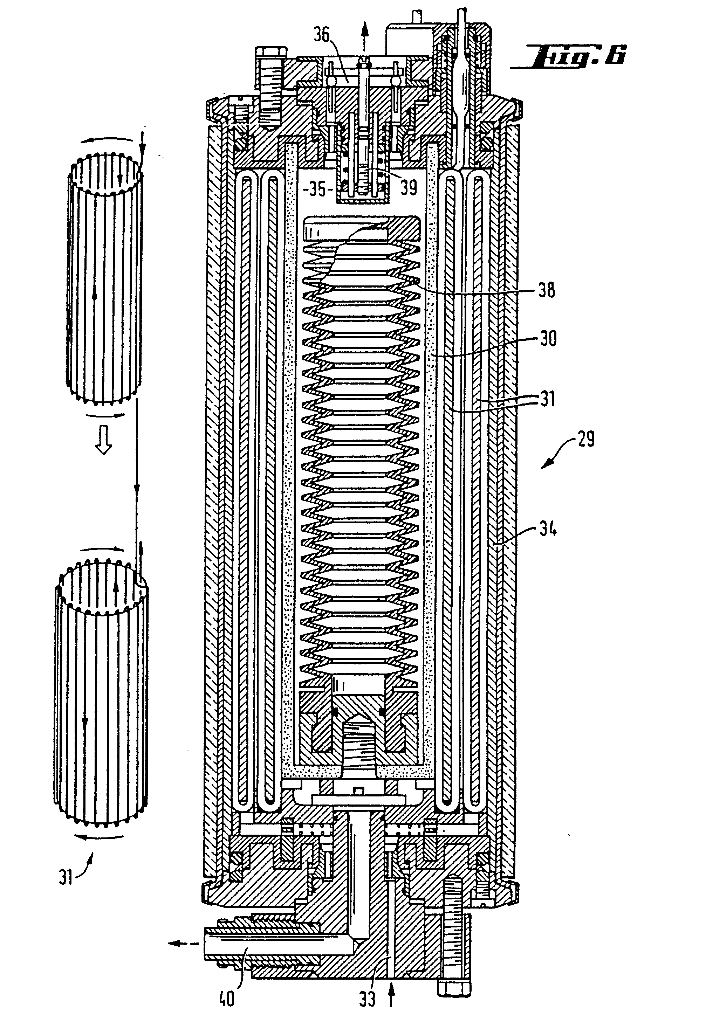

Die einzelnen Membrantaschen des Spiralwickelmoduls sind durch Abstandsfolien voneinander getrennt, so dass zwischen den einzelnen gewickelten Lagen der Membrantaschen ausreichend Abstand zum Durchströmen des druckbeaufschlagten Fermentationsgemisches gegeben ist. Dabei ist das Spiralwickelelement an seinen beiden Stirnseiten durch Abschlussplatten 94, 95 hermetisch abgedichtet. Auf diese Weise wird bewirkt, dass das unter Druckbeaufschlagung durch eine zentrale Zuleitungsbohrung 96 im Wickelkern 93 eintretende und durch radiale Kanäle 97 in den Beginn der spiraligen Zwischenräume zwischen den aufgewickelten Membranen geleitete Fermentationsgemisch das Spiralwickelelement tangential von radial innen nach radial aussen durchströmt. Dabei wird zwischen den Membranwicklungen, die in der Fig. 4 schematisch dargestellt sind, eine dünne Strömungsschicht, in der Regel bei geeigneter Prozessführung und geeigneter Wicklung ein dünner Strömungsfilm des Fermentationsgemisches auf den Membranoberflächen ausgebildet. Das am radialen Aussenrand des Spiralwickelmoduls tangential auftretende durchgedrückte Fermentationsgemisch läuft in einem Ringspalt zwischen dem Aussenrand des Spiralwickelelements und der zylindrischen Innenwand des Reaktorgefässes 57 auf den Boden des Reaktorgefässes 57 ab, auf dem ein Speicher- und Steuerreservoir des umgewälzten Fermentationsgemisches gehalten wird. Dabei wird das Niveau dieses Reservoirs durch einen Schwimmer 56 gesteuert, der unmittelbart ein Stellventil 55, hier ein Schlauchknickventil, stellt, das in der Zuleitung 54 für das frische sterile Substrat liegt. Auf diese Weise wird der Zulauf an frischem sterilen Substrat stets automatisch nach Massgabe der durch die Membran hindurch abgetrennten flüssigen Phase ergänzt.

Über die Sterildruckluftleitungen 81, 82, in denen jeweils separate Einstellventile 79, 80 liegen, können die Ableitungsröhrchen 89 bis 92 auch mit steriler Druckluft beaufschlagt werden. Die Druckluft kann auf diese Weise in die im Spiralwickelelement aufgewickelten Membrantaschen eintreten und dann je nach Druckregelung entweder durch die Membran hindurch in den Gasraum des Reaktorgehäuses 57 eintreten und dort beispielsweise bei Inbetriebnahme des Reaktors die Membran trockendrücken, oder kann bei geringerem Luftdruck der Rückspülung der Membrantaschen dienen. In gleicher Weise kann das Spiralwickelelement auch so ausgelegt sein, dass stets eine bestimmte Anzahl von Membrantaschen der filtrierten Zufuhr entkeimter Luft in den Druckgasraum des Reaktors dient, während die verbleibenden Membrantaschen der Produktabtrennung, also dem Mediumtransport in entgegengesetzter Richtung, dienen. Bei entsprechender Anordnung der Leitungen und Ventile kann der Membranreaktor auch in dieser Betriebshinsicht frei programmierbar ausgelegt sein. Unabhängig davon jedoch, wie der Membranreaktor nun im einzelnen ausgelegt ist, bleibt an dem im Rahmen des Verfahrens und der Vorrichtung der Erfindung eingesetzten Membranreaktor wichtig, dass das Fermentationsgemisch unter Druck in einer dünnen Schicht über eine Membranoberfläche geleitet wird, dabei durch intensiven Stoffaustausch hochwirksam fermentiert werden kann und sich unter optimalen Bedingungen homogen entwickeln kann, während gleichzeitig die im fermentierten Gemisch enthaltenen Stoffwechselprodukte kontinuierlich über Filtratsammelleitungen aus dem Reaktor abgezogen werden können, während das Fermentationsgemisch ohne jeglichen Verlust an Mikroorganismen oder anderer Biomasse in einem geschlossenen Kreislauf stets von neuem über die Membranoberfläche unter kontinuierlichem Zusatz von Substrat und Luft oder Luft/Sauerstoffzusätzen geführt wird.

Zur Belüftung ist der Membranreaktor 58 am Kopf des Reaktorgehäuses 57 mit einem Luftfilter 98 versehen, dem ein einstellbares Sicherheitsbelüftungsventil 99 nachgeschaltet ist. Über dieses Ventil 99 gelangt die Abluft aus dem Membranreaktor 58 in eine Abluftleitung 100. Auch der Abluftfilter 98 weist in dem hier gezeigen Ausführungsbeispiel wiederum eine hydrophobe PTFE-Membran auf.

**Patentansprüche**

1. Vorrichtung zur kontinuierlichen Fermentation mit gleichzeitiger fraktionierter Stoffwechselproduktabtrennung, gekennzeichnet durch einen als geschlossenen Druckbehälter ausgebildeten Reaktor, in dem ein Membranfilter-Spiralwickelelement angeordnet ist, bei dem auf einem Wickelkern mindestens eine taschenförmig ausgebildete Membran unter Zwischenlage mindestens einer Abstandshalterfolie in der Weise aufgewickelt ist, dass jede Membrantasche einen eigenen separaten Ablauf zu einem eigenen, separaten axial im Kern verlaufenden Ablaufrohr aufweist, das von ausserhalb des Reaktors über Anschlüsse zugänglich ist, und bei dem im Wickelkern eine zentrale Zulaufleitung für das Fermentationsgemisch ausgebildet ist, die durch radiale Kanäle oder Öffnungen, die über die gesamte axiale Länge des Wickelkerns gleichmässig verteilt sind, zum Aussenmantel des Wickelkerns offen ist, dessen Stirnseiten in der Weise dicht durch Abdeckungen verschlossen sind, dass das in die zentrale Zulaufleitung eingedrückte Fermentationsgemisch das Spiralwickelelement in tangentialer Richtung von radial innen nach radial aussen in dünner Schicht durchströmt und dann nach tangentialem Austritt aus dem Spiralwickelelement auf den Boden des Reaktors abfliessen kann, von wo eine Kreislaufleitung, in die ein Drosselventil und eine Druckpumpe eingeschaltet sind, zur zentralen Zulaufleitung im Wickelkern des Membranfilter-Spiralwickelelements zurückgeführt.

2. Vorrichtung nach Anspruch 1, gekennzeichnet durch eine Sterildruckluftleitung, die über ein Stellventil mit den Ablaufröhrchen der Membrantaschen des Spiralwickelmoduls verbindbar ist.

3. Vorrichtung nach Anspruch 1, gekennzeichnet durch einen Sensor, der das Niveau der Flüssigkeit über dem Boden des Reaktors abtastet und nach Massgabe des abgetasteten Niveaus ein Ventil stellt, das in einer Zulaufleitung liegt, die die Kreislaufleitung des Fermentationsgemisches mit einem Substratvorratsspeicher verbindet.

4. Vorrichtung nach Anspruch 3, dadurch gekennzeichnet, dass der Substratvorratsspeicher eine Ringleitung ist, in der ein Vorratsbehälter eingeschlossen ist, und in der das vorfiltrierte und vorgewärmte Substrat unter Temperaturregelung im Kreislauf geführt wird, und dass von der Ringleitung eine Verbindungsleitung abzweigt, die in die Zulaufleitung zur Kreislaufleitung des Fermentationsgemisches einmündet und in der ein mit einer Heizung und einem Filter ausgestattetes Sterilisierungsmodul liegt.

5. Vorrichtung nach Anspruch 4, gekennzeichnet durch einen Wärmetauscher, der warmseitig nach dem Sterilisierungsmodul in der Verbindungsleitung und kaltseitig in der Substratringleitung liegt.

6. Vorrichtung nach Anspruch 1, dadurch gekennzeichnet, dass die Membran des Membranfilter-Spiralwickelelements hydrophob ist.

**Claims**

1. A device for continuous fermentation accompanied by simultaneous fractionated metabolic product separation, characterized in that there are provided: a reactor built as a closed pressure vessel, in which is arranged a membrane filter-spiral winding element, at which is wound up on a winding core at least a pocket-shaped membrane by intercalating an intermediate layer at least a spreader foil such that each membrane pocket comprises an own separate draining means to an own separate discharging pipe axially arranged to said core, which is accessible from an outside position of said reactor by means of connections, and at which a central supply pipe is formed within said winding core for the fermentation mixture, which is opened by radial channels or openings equally distributed over the total axial length of said winding core, to an outer casing of said winding core, the front faces of which are tightly closed such that said fermentation mixture pressed into said central supply pipe is flowing through said spiral winding element in tangential direction from a radial inside to a radial outside position as a thin layer and then, following tangential emergence from said spiral winding element, may flow off to the bottom of said reactor, from where a circular conduit pipe, into which is inserted a flow control valve and a pressure pump, will lead back to said central supply pipe within said winding core of said membrane filter-spiral winding element.

2. The device as claimed in claim 1, characterized in that there is provided a sterile compressed-air piping which is connectable via an actuator to said draining tubes of said membrane pockets of the spiral winding module.

3. The device as claimed in claim 1, characterized by a sensor scanning the level of the fluid above said bottom of said reactor and actuating a valve in accordance with said scanned level, whereby the valve is situated within a supply pipe connecting said circulation conduit pipe of said fermentation mixture with a substrate supply storage.

4. The device as claimed in claim 3, characterized in that the substrate supply storage is a circular pipeline, in which a supply vessel is enclosed and in which the pre-filtered and pre-heated substrate is conducted by temperature control in said circulation means, and that from said circular conduit pipe a connecting pipe branches off merging into said supply pipe of the circular conduit pipe of the fermentation mixture and within which is situated a sterilizing module equipped with a heating and a filter means.

5. The device as claimed in claim 4, characterized in that there is provided a heat exchanger which, on the hot side, is situated in the vicinity of said sterilizing module within said connecting pipe and, on the cold side, within said substrate circular conduit pipe.

6. The device as claimed in claim 1, characterized in that said membrane of said membrane filter-spiral winding element is hydrophobic.

**Revendications**

1. Dispositif pour la fermentation continue et la séparation fractionnée de produits du métabolisme, caractérisé par un réacteur qui est constitué par un réservoir fermé sous pression contenant un élément d'enroulement en spirale pour filtre à membrane dans lequel au moins une membrane en forme de poche est enroulée sur un axe de bobinage, avec interposition d'au moins une feuille de maintien à distance, de telle manière que chaque poche de membrane comporte une sortie propre séparée dans un tube de sortie propre séparé orienté axialement dans le noyau et accessible de l'extérieur du réacteur par des raccords, et dans lequel le noyau d'enroulement comporte un conduit central pour l'amenée du mélange de fermentation qui, par des conduits ou des ouvertures orientés dans le sens radial et répartis régulièrement sur toute la longueur de l'axe de bobinage, débouchent sur l'enveloppe extérieure de l'axe de bobinage dont les côtés frontaux sont hermétiquement fermés par des dispositifs de recouvrement de telle manière que le mélange de fermentation introduit sous pression dans le conduit d'amenée central suit l'élément en spirale, en couche mince, dans le sens tangentiel de l'intérieur vers l'extérieur dans le sens radial et peut ensuite, après une sortie tangentielle hors de l'élément d'enroulement en spirale, s'écouler sur le fond du réacteur d'où un conduit de circulation qui comprend une soupape d'étranglement et une pompe refoulante, le ramène au conduit d'amenée central ménagé dans l'axe de bobinage de l'élément d'enroulement en spirale pour filtre à membrane.

2. Dispositif selon la revendication 1, caractérisé par un conduit à air comprimé stérile qui peut être relié par une soupape de réglage aux petits tubes de sortie de poches à membranes du module d'enroulement en spirale.

3. Dispositif selon la revendication 1, caractérisé par un organe sensible qui détecte le niveau du liquide au-dessus du fond du réacteur et, après mesure du niveau détecté, règle une soupape située dans un conduit d'amenée qui relie le conduit de circulation du mélange de fermentation à un réservoir de substrat.

4. Dispositif selon la revendication 3, caractérisé en ce que le réservoir de substrat est un conduit annulaire auquel est incorporé un réservoir et dans lequel le substrat préalablement filtré et chauffé suit un circuit comportant un réglage de la température et en ce que le conduit annulaire est relié à un conduit de liaison qui débouche dans le conduit d'amenée aboutissant au conduit de circulation du mélange de fermentation et qui contient un module de stérilisation équipé d'un dispositif de chauffage et d'un filtre.

5. Dispositif selon la revendication 4, caractérisé par un échangeur de chaleur qui se trouve du côté chaud dans le conduit de liaison après le module de stérilisation et du côté froid dans le conduit annulaire à substrat.

6. Dispositif selon la revendication 1, caractérisé en ce que la membrane de l'élément d'enroulement en spirale de filtre à membrane est hydrophobe.

Fig. 1

0 150 197

## Legenden zu Figur 1

| ISO Kurz-bezeichnung | Benennung |
|---|---|
| F 1 - Y 1 | Teflonmembran-Luftfilter mit Sicherheitsventil |
| F 2 | Poröse Teflon-Vorfilter |
| H 3 | Zwei-Wege-Kugelhahn |
| H 4 | Zwei-Wege-Kugelhahn für Kühlanschluß |
| B 5 - LI 5 | Wärmeisolierter Zirkulationsbehälter mit Niveauanzeige und Kühlspirale |
| TPI 6 | Thermomanometer |
| TE 7 - VTE 7 | Thermofühler mit Thermostatventil |
| H 8 | Drei-Wege-Kugelhahn |
| P 9-M9 - R9 | Druckerhöhungspumpe mit Motor und Einstellventil |
| PEI 10 | Druckaufnehmer mit Überwachung und Anzeige |
| R 11 | Rückschlagventil |
| R 12 | Einstellbares Sicherheits-Rückschlagventil |
| H 13 | Drei-Wege-Kugelhahn |
| F 14 - VTE 14 -B 14 | Wärmeisolierter, beheizbarer, poröser Teflonfilter mit Thermostatsicherung und Verweilzeitbehälter |
| R 15 | Einstellbares Sicherheits-Rückschlag-ventil |
| R 16 | Rückspülbehälter |
| VP 17 | Plunger-Schlauchventil |
| TEI 18 | Temperaturaufnehmer mit Regelung und Anzeige |
| TPI 19 | Thermomanometer |
| R 20 | Rückschlagventil |
| W 21 | Wärmeaustauscher für Vorsterilisieren |
| R 22 | Rückschlagventil |
| W 23 | Wärmeaustauscher für Temperieren |
| TPI 24 | Thermomanometer |
| H 25 | Zwei-Wege-Kugelhahn |
| VTE 26 - TE 26 | Thermostatventil mit Thermofühler |
| TPI 27 | Thermomanometer |
| R 28 | Einstellbares Rückschlagventil |
| TPI 29 | Thermomanometer |
| R 30 | Einstellbares Rückschlagventil |
| FI 31 | Durchflußmesser für Substrat |

11

# Fig:2

0 150 197

## Legenden zu Figur 2

| ISO Kurz-bezeichnung | Benennung |
|---|---|
| MBR 32 - X 32 | Membranbioreaktor mit Schwimmerventil |
| H 33 | Zwei-Wege-Kugelhahn - Drosselarmatur |
| P 34 - M 34 | Zirkulationspumpe |
| TPI 35 | Thermomanometer |
| R 36 | Einstellbares Rückschlagventil |
| H 37 | Drei-Wege-Kugelhahn |
| P 38 | Injektorpumpe |
| R 39 | Rückschlagventil |
| PI 40 | Manometer |
| FI 41 | Durchflußmesser (für Luft) |
| H 42 | Entkeimungsfilter-Block |
| H 43.1 | Dreiwege-Kugelhahn-Block |
| H 44 | Drei-Wege-Kugelhahn |
| R 45 | Einstellbares Rückschlagventil |
| H 46 | Beheizbarer Zwei-Wege-Kugelhahn |
| R 47 | Rückschlagventil |
| P 48 - M 48 | Rotationskompressor |
| PEI 49 | Druckaufnehmer mit Überwachung und Anzeige |
| V 50 | Druckdifferenz-Regelventil |
| R 51 | Einstellbares Rückschlagventil |
| FI 52 | Durchflußmesser für Luft |
| FI 53 | Durchflußmesser für Luft |
| R 54 | Einstellbares Rückschlagventil |
| R 55 | Einstellbares Rückschlagventil |
| H 56 | Drei-Wege-Kugelhahn |
| H 57 | Drei-Wege-Kugelhahn |
| FI 58 | Durchflußmesser für Fraktion |
| FI 59 | Durchflußmesser für Fraktion |
| R 60 | Einstellbares Rückschlagventil |
| R 61 | Einstellbares Rückschlagventil |
| F 62 - Y 62 | Abluftfilter mit Sicherheitsventil - einstellbares Rückschlagventil |
| FI 63 | Durchflußmesser (für Abluft) |

15

*Fig.3*

# Fig. 4

# Fig. 5

**Fig. 6**